# EUROPEAN PATENT SPECIFICATION

(11) **EP 2 875 805 B1**
(45) Date of publication and mention of the grant of the patent: **28.06.2017**
(21) Application number: 13194510.7
(22) Date of filing: 26.11.2013
(51) Int. Cl.: A61K 8/64, A61Q 19/08, A61K 8/99

(54) **Method for the isolation of glycoprotein-rich fungal extract and its use in anti-ageing cosmetic formulations**
Verfahren zur Isolierung von glycoproteinreichen Pilzextrakten und deren Verwendung in kosmetischen Formulierungen gegen Hautalterung
Procédé pour l'isolement d'extrait fongique riche en glycoprotéine et son utilisation dans des formulations cosmétiques anti-âge

(43) Date of publication of application: 27.05.2015
(73) Proprietor: Latvijas Universitate, 1586 Riga (LV)
(72) Inventor: Ramata-Stunda, Anna, LV-1011 Riga (LV); Nikolajeva, Vizma, LV-1586 Riga (LV); Petrina, Zaiga, LV-1007 Riga (LV); Cakstina, Inese, LV-1083 Riga (LV); Grine, Lita, LV-1010 Riga (LV); Blake, Ilze, LV-1024 Riga (LV); Muiznieks, Indrikis, LV-1011 Riga (LV)
(74) Representative: Fortuna, Aleksandra

(56) References cited:
- LV-B- 13 959
- Vizma Nikolajeva ET AL: "Treatment of skin ulcers with adenylate deaminase, a glycoprotein from microscopic fungus Penicillium lanoso-viride", Acta Universitatis Latviensis, 1 January 2009 (2009-01-01), pages 69-79, XP055114984, Latvia Retrieved from the Internet: URL:http://eeb.lu.lv/EEB/2009/Nikolajeva.p df [retrieved on 2014-04-23]
- VIZMA NIKOLAJEVA ET AL: "Protective effect of adenylate deaminase (from Penicillium lanoso-viride) against acute infections in mice", IMMUNOPHARMACOLOGY, vol. 35, no. 2, 28 March 1996 (1996-03-28) , pages 163-169, XP055114912, ISSN: 0162-3109, DOI: 10.1016/S0162-3109(96)00142-7
- J K Thakkar ET AL: "Cardiac adenylate deaminase: molecular, kinetic and regulatory properties under phosphate-free conditions", The Biochemical journal, 1 June 1994 (1994-06-01), pages 359-363, XP055115066, ENGLAND Retrieved from the Internet: URL:http://www.pubmedcentral.nih.gov/artic lerender.fcgi?artid=1138170&tool=pmcentrez &rendertype=abstract
- MAKAREWICZ ET AL: "AMP-aminohydrolase in muscle of elasmobranch fish. Purification procedure and properties of the purified enzyme", COMPARATIVE BIOCHEMISTRY AND PHYSIOLOGY, PERGAMON, vol. 29, no. 1, 1 April 1969 (1969-04-01), pages 1-26, XP025476642, ISSN: 0010-406X, DOI: 10.1016/0010-406X(69)91723-X [retrieved on 1969-04-01]

## Description

### Technical field

The present invention relates to a method of isolation of glycoprotein-rich fungal extract, according to claim 1, and production of anti-ageing and skin regenerating formulations containing fungal glycoprotein extract and its combinations with plant extracts.

### Background art

One of the most frequent cosmetological concerns is skin ageing, which is an evitable process. Research during previous decades has revealed different causes and mechanisms of skin ageing, which in turn allows to search for active ingredients effective against specific ageing mechanisms. Consumers are particularly demanding effective natural anti-ageing skin care products and that stimulates extensive research of plant and fungi derived active active ingredients (Jenkins, 2002, Robert et al., 2009).

Natural ingredients used in cosmetic products contain a variety of preparations or isolated active substances. Preparations include plant juices, extracts, lipids, polysaccharides. Among purified active substances there are vitamins and other ingredients. There is a wide range of food plants (wild and cultivated), medicinal fungi, herbs and naturally occurring microorganisms that are used as sourced of cosmetic ingredients. (Pieroni et al, 2004, Antignac et al., 2011, Mukherjee et al., 2011, Yingngam, Rungseevijitprapa, 2012).

Skin suffers progressive morphologic and physiologic decrements with time. Exposed areas of the body, such as the face, the neck, and the hands, suffer the most by the influence of extrinsic factors and overexposure of these regions may result in prematurely aged skin. Solar irradiation in the form of ultraviolet (UV) radiation and tobacco smoking are by far the most important environmental factors. With accelerating age skin loses its structural and morphologic characteristics, and its functions deteriorate as a consequence With time, the epidermis develops an abnormality in permeability barrier homeostasis that is accentuated further in photoaged skin. Consequently, skin becomes more susceptible to mechanical trauma and infections. In addition, balances of the serum levels of sex steroids, which naturally occur at menopause in women, may result in changed skin structure and functionality (Zouboulis and Makrantonaki, 2011).

Ageing of the skin is commonly associated with increased wrinkling, sagging and increased laxity, but when considering the underlying reasons for these changes, it is important to distinguish between the effects of intrinsic biological ageing and environmental factors, such as exposure to the sun. There is also a reduction in the number and biosynthetic capacity of fibroblasts (Bolognia, 1993) and progressive disappearance of elastic tissue in the papillary dermis. Skin collagen content decreases with age and the fine collagen fibres become increasingly dense and tightly packed and more randomly orientated The processes associated with intrinsic skin ageing are thought to result from a combination of events including decreased proliferative capacity of skin derived cells; decreased synthesis of the extracellular matrix components in the dermis; and increased expression of enzymes that degrade the collagenous matrix. Ageing is heavily influenced by external oxidative stresses. Oxidative damage as a causative factor in ageing is supported by a body of experimental findings (Sohal and Weindruch, 1996; Guyton et al., 1997). It is particularly relevant in skin ageing given its high exposure to environmental agents such as ultraviolet radiation and ozone. Reactive oxygen species cause damage to lipids, proteins and DNA and also influence cellular senescence. It has been suggested that as much as 80% of facial ageing is attributable to sun exposure (Gilchrest, 1989, Campisi, 1998).

Fungal and plant polysaccharides and glycoproteins are used in cosmetics as well as wound healing. Amongst polysaccharides β-glucans (polysaccharides of D-glucose monomers linked by β-glycosidic bonds) are the most widely used and best characterized. β-glucans are widely used not only as nutritional supplements or oral medicines but also cosmetic active ingredients. It has been reported that β-glucans besides their immunomodulatory characteristics have additional antioxidant properties. Because oxidative stress is one of the major reasons for skin ageing and dermatological conditions, phytochemicals with proven antioxidant activity are useful for anti-ageing cosmetics products and skin treatment of several dermatologic conditions (Singh and Agarwal, 2009, Du et al., 2013). Some authors claim that β-glucans possess also anti-wrinking activity. Formation of wrinkles is due to decline of collagen in dermal layed of aged skin, so it is speculated that □-glucans anti-wrinkling activity is most probably due to promotion of collagen synthesis of dermal cells. It has been shown that fungal polysaccharides, with β-glucans being amongst them, boost keratinocyte cell growth against skin ageing by altering gene expression (Saoquing et al., 2008). β-glucans and other polysaccharides play important role in cosmetic industry as components of skin moistorizers. Contrary to the common belief that large biopolymers find it difficult to penetrate deeply in the skin, when applied topicaly, it has been proved that b-glucans despite their large molecular masses penetrates the skin in to dermis. It does not penetrate cells but penetrates dermis through intercellular space (Du et al., 2013). Patent application WO 2007062995 A3 claims use of glucan in skin moisturizing, anti-ageing and revitalizing formulations.

Glycoproteins are less characterized group of natural biopolymers than polysaccharides in context of cosmetics and skin care, however interest about them as active ingredients is increasing.

There are several examples of use of glycoprotein isolated from arctic bacteria as cosmetic ingredient. A skin tightening cosmetic composition described in patent application WO 2008084890 A1 contains a glycoprotein, preferably *Pseudoalteromonas antarctica* NF3. The glycoprotein used is described to have ability to stimulate cell growth, prevent skin drying and protect epidermis. Patent US 7022668 B2 claims use of glycoprotein produced by *Pseudoalteromonas antartica* which consists of 14% carbohydrates and 86% protein in cosmetic formulations. It is shown in the description of invention that glycoprotein used in cosmetic composition promotes keratinocyte proliferation and adhesion.

Patent application WO 1996020284 A1 claims use of hydroxyproline-rich glycoproteins, obtainable by acid-alcohol extraction from *Taxus* spp., *Ginko biloba, Lycopersicum esculentum* and *Daucus carota* cell cultures, in hydrating, film-forming, toning and cicatrizant cosmetic products.

In patent EP 0668072 B1 an invention of a new cosmetic composition comprising a plant extracellular matrix extract, comprising a glycoprotein in substantially native conformation, a carbohydrate polymer in substantially native conformation and an arabinogalactan protein in substantially native conformation and a cosmetic carrier is described. Invented composition is targeted to repair and remodeling of a dermo-epidermal interface damaged by factors such as aging or exposure to environmental factors such as ultraviolet light.

Use of fungal glycoprotein adenylate deaminase (AMPD) containing hydrogel for treatment of skin ulcers has been described in inventors' earlier patent LV13959. AMPD used for production of hydrogel in this patent was isolated from laboratory strain of soil fungus *Penicillium lanoso-viride* 8D.

Previous studies have shown that AMPD isolated from *P.lanoso-viride* 8D has immunomodulatory characteristics - it modulates both cellular and humoral immunity. AMPD promotes proliferation of lymphocytes, activates murine peritoneal macrophages (Nikolajeva et al., 1999), stimulates natural killers and possesses antitumor activity (Zak et al., 1986), enhances resistance to infections (Nikolajeva et al., 1996), inhibits experimental autoimmune encephalomyelitis (Nikolajeva et al., 2000).

Current experience of glycoprotein use in cosmetics together with known biological activities *P.lanoso-viride* 8D glycoprotein AMPD have been preconditions for us to develop simplified glycoprotein isolation method and use of extracted active substances in skin regenerating cosmetics.

### Description of the invention

The present invention is related to fast and cost-effective extraction of glycoprotein rich fraction from *Penicillium lanoso-viride* 8D, according to claim 1, and use of it in skin regenerating cosmetic composition. For use in cosmetic purposes, a simplified production method of glycoprotein enriched fraction was developed, avoiding gel filtration. Additionally use of purified *P.lanoso-viride* 8D glycoprotein fraction possessing AMPD activity for cosmetic purposes was developed.

There is offered a method, according to claim 1, for the isolation of glycoprotein-rich fungal extract from sporulating mycelium of fungus *Penicillium lanoso-viride* 8D, cultured on the surface of malt extract broth, by disintegration and extraction of mycelium in phosphate buffered saline with following enrichment. The enriched glycoprotein fraction is isolated by precipitation at 4 °C with ammonium sulphate at 50-77% saturation. The purified, adenylate deaminase activity possessing glycoprotein fraction is isolated with gelfiltration of enriched glycoprotein fraction on Sephadex G-150 or G-200 with phosphate buffered saline. The eniched glycoprotein fraction can be used in cosmetic compositions at concentrations 5-15 mg/ml for skin regenerating purposes. The purified glycoprotein fraction can be used in cosmetic compositions at concentrations 5-10 µg/ml for skin regenerating purposes.

### Brief Description of figures:

Fig. 1. Effect of purified glycoprotein fraction (AMPD) on scratch wound closure in dermal fibroblast cell culture within 24h of cultivation in presence of AMPD.
Fig 2. Effect of purified glycoprotein fraction (AMPD) on scratch wound closure in HaCaT keratinocyte culture within 24h of cultivation in presence of AMPD.
Fig 3. Effect of purified glycoprotein fraction (AMPD) on population doubling time in dermal fibroblast cell line at 24, 48, 72 and 96h of cultivation in presence of AMPD at indicated concentrations.
Fig. 4. Effect of purified glycoprotein fraction (AMPD) on population doubling time in HaCaT keratinocyte line at 24, 48, 72 and 96h of cultivation in presence of AMPD at indicated concentrations.
Fig.5. Effect of purified glycoprotein fraction (AMPD) on expression of collagen I, elastin and MMP-1 genes after cultivation for 7 days in presence of AMPD.

Production of glycoprotein enriched fraction. For isolation of glycoprotein rich fraction *Penicillium lanoso-viride* 8D was cultivated on the surface of malt extract broth (30g/L malt extract) at room temperature. As production of previously characterized AMPD is most pronounced in *P.lanoso-viride* 8D at sporulation stage, mycelia for extraction of glycoproteins were collected by filtration on 5^{th} day of cultivation.

Mycelium was ground in sterile phosphate buffer solution (PBS) (pH 7.4) in bead mills (speed 700 rpm, for total of 15 minutes with intermittent cooling) and homogenate was centrifuged for 40 min at 4 °C at speed 4000 x g. Supernatant was collected for glycoprotein precipitation. Supernatant was fractioned by stepwise ammonium sulphate precipitation:
1) 33 g of ammonium sulphate was slowly added per 100 ml of extract (50% saturation of ammonium sulphate at 4 °C) followed by centrifugation for 40 minutes at 4 °C at speed 4000 x g; pellet was discarded and supernatant used for further precipitation;
2) 22 g of ammonium sulphate is added to supernatant (77% saturation of ammonium sulphate at 4 °C) followed by centrifugation for 40 minutes at 4 °C at speed 4000 x g; supernatant was discarded and pellet resuspended in PBS

Dialysis of suspension against 200 volumes of PBS was performed overnight. Protein concentration in samples was determined spectrophotometrically (A₂₈₀ₙₘ). Acquired concentrations were in range 20 - 40 mg/ml. For use in cosmetic formulations 5-15 mg/ml glycoprotein crude extract were used, formulations of products containing glycoprotein are described in examples 1-3 given below.

Production of purified glycoprotein fraction possessing AMPD enzymatic activity. For isolation of purified fraction glycoproteins were enriched from *P.lanoso-viride* 8D as described above. After dialysis, enriched fraction was further fractioned by gel filtration on Sephadex G-200 column (dimensions 2.5 x 90 cm). High molecular weight glycoprotein fractions are eluted from the column with PBS at flow rate 0.25 - 0.30 ml/min.

AMPD activity, characteristic to purified glycoprotein fraction, was determined in each collected fraction - one unit of enzymatic activity was determined as quantity of AMPD that deaminated 1 µmol of 5'-AMP to 5'-IMP per minute at 37 °C (pH 6.0). (Nikolajeva et al., 1996). Protein concentrations of the fractions were determined spectrophotometrically. Isolation yields 0.5-1.5 mg/ml protein. For use in cosmetic formulations 5-10 µg/ml purified glycoproteins are used. Formulations of products containing purified glycoprotein fraction are described in examples 4-5.

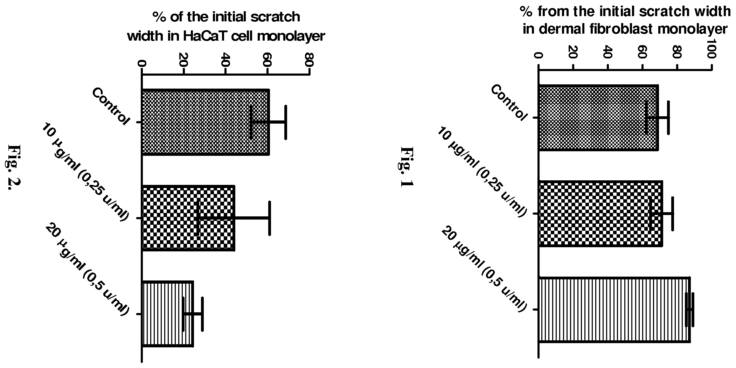

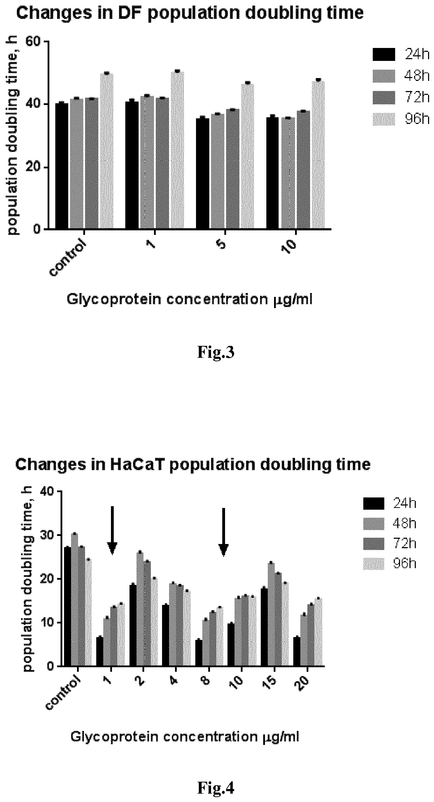

Biological activity of purified glycoprotein fractions was tested *in vitro* in skin cell cultures:
1) Evaluation of purified glycoprotein's (AMPD) effect on cell proliferation. Scratch tests in cell monolayer cultures in presence of AMPD were performed to assess effect of purified glycoprotein fraction on cell proliferation and migration. In short 24-well plates were used for scratch assays. 2.5 x 10⁴ dermal fibroblasts or HaCaT keratinocytes were seeded per cm² of cultivation surface. Cells were grown in 10% FBS/DMEM medium (Dulbeco's modified Eagle's medium, supplemented with 10% fetal bovine serum and 10 u/ml penicilline, 100 µg/ml streptomycine) for 24 to 48 hours to reach 100% confluence. A sterile 1000 µl pipette tip was used to introduce a scratch "wound" on cell monolayer of each well. After scratching each well was rinsed with PBS. Media supplemented with AMPD fraction were added to scratched wells. Each well was photographed at 10x objective magnification at defined time points - 0h and 24h. Results of three independent experiments are shown in Fig 1. for dermal cells and Fig.2 for HaCaT keratinocytes.
   Additionally effect of AMPD fraction on proliferation of skin cells was tested using real-time cell monitoring system. The cell proliferation in the presence of fraction was monitored using xCELLigence™ System (Roche, USA). Cells were seeded on xCelligence system compatible E-plate at a density 1000 cells per well and allowed to attach for 24 h before supplementing with test fraction. Cells were cultivated for 96 h after adding the glycoprotein fraction. Arbitrary cell index values were measured every 30 minutes of cultivation and normalized to the time point of the addition of glycoprotein fraction. The population doubling times were calculated by RCTA v1.2 software after 48 h, 72 h and 96 h of cultivation. Effect of purified fraction on cellular proliferation is shown in Fig 3. for dermal cells and Fig.4 for HaCaT keratinocytes.
   Both methods, scratch test and real-time cell proliferation monitoring, showed consistent results
   - purified fungal glycoprotein fraction stimulates proliferation of keratinocytes, however it does not exert stimulatory effect on dermal cells. Stimulation of keratinocyte proliferation was time dependent - within the first 24h after addition of glycoprotein fraction stimulatory effect is most pronounced. Stimulation is not linearly dose dependent - both low concentrations (1 µg/ml) and higher (8-10 µg/ml) reduce population doubling time of keratinocytes. *In vitro* testing results show that purified glycoprotein fraction is potent skin regenerating and anti-ageing active ingredient as it stimulates epidermal cell proliferation.
2) Evaluation of purified glycoprotein's (AMPD) effect on gene expression of dermal structural proteins and matrix metalloproteinase 1. To evaluate effect of glycoprotein in structural protein gene expression, dermal fibroblasts are seeded in 24-well plates in 10% FBS/DMEM medium (Dulbeco's modified Eagle's medium, supplemented with 10% fetal bovine serum and 10 u/ml penicillin, 100 µg/ml streptomycin). After addition of glycoprotein at concentration 10 µg/ml dermal fibroblasts are cultivated for 7 days. To isolate RNA from cells treated with AMPD cell cultivation media was aspirated and cultivation plates were rinsed twice with PBS. 500 µl of Trizol LS reagent was added to each well of 24-well cultivation plate. After 5 minutes Trizol LS reagent was aspirated in sterile Eppendorf microcentrifuge tubes. Samples were extracted by addition of 200 µl of chloroform and incubation at room temperature for 10 minutes. After centrifugation at 12 000 x g at 4 °C aqueous RNA containing phase was transferred to new microcentrifuge tube and precipitated with one volume of isopropanol. Mixture was centrifuged at 12 000 x g for 10 minutes and washed afterwards with ice cold 70% ethanol. RNA pellet was suspended in DEPC-treated water. Concentrations and quality of RNA were evaluated spectrophotometrically and by agarose gel electrophoresis. Isolated RNA was DNaseI treated for 30 minutes at 37 °C to remove residual DNA. cDNA was synthesized using commercially available First strand cDNA synthesis Kit.

50 ng cDNA was used as a template in qPCR reactions with oligonucleotides specific for the genes of interest - collagen I, elastin, matrix metalloprotease 1 (MMP-1). Quantification was done by measuring SYBR green fluorescence on Applied Biosystems 7300 amplificator. Human beta-actin gene was employed as a reference house-keeping gene and 2-ΔΔCt method used to quantify relative changes in gene expression (Livak and Schmittgen 2001). Relative gene expression level of control samples was set to 100% and results for AMPD treated samples were compared to it.

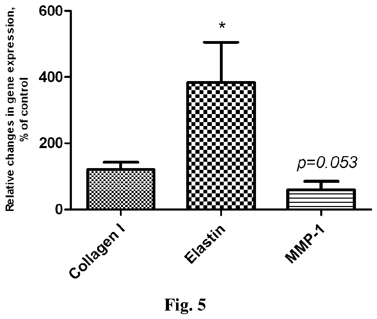

Effect of glycoprotein on expression of collagen I, elastin and MMP-1 is shown in Fig 5. It was observed that AMPD fraction induces expression of collagen I gene - increase by 20% compared to control, and elastin gene expression - increase by 284% (2.84 fold increase) compared to control and suppression of MMP-1 expression - decrease by 40.60 %. Data show that fungal glycoprotein fraction is potent stimulator of extracellular matrix components, moreover it downregulates expression of collagen I degrading enzyme MMP-1. This in turn indicates on potential of tested fraction to be included in skin regenerating and anti-ageing cosmetic formulations.

### Examples of implementation of the invention

**Example 1 - regenerating cream containing glycoprotein enriched fraction from P.lanoso-viride8D**

| | | |
|---|---|---|
| a) | Aqua | 54,45% |
| b) | Vitis vinifera (Grape) Seed Oil | 20% |
| | Caprylic/capric triglycerides | 10% |
| | Titanium dioxide | 6% |
| | Olivem-100 (Cetearyl Olivate, Sorbitan Olivate) | 4% |
| | Cetyl alcohol | 2% |
| | Enriched *P. lanoso-viride* 8D glycoprotein fraction | 1,5% (10 mg/ml) |
| | Ethanol | 0,75% |
| | Tocopherol acetate | 0,5% |
| | Potassium sorbate | 0,4% |
| | Gluconolactone, Sodium Benzoate | 0,4% |

| | | |
|---|---|---|
| Ingredient a) is heated to approximately 65°C b) is likewise heated to approximately 65 °C till emulsifying waxes are completely melted, and then added while vigorously stirring to mixture a) to form emulsion. Stirring is continued until the cream has cooled down to approximately 35 °C. Then mixture c) is added while stirring and the cream is homogenized. | | |

**Example 2 - facial cleansing milk containing glycoprotein enriched fraction from P.lanoso-viride 8D**

| | | |
|---|---|---|
| a) | Aqua | 76,45% |
| | Xanthan gum | 4% |
| b) | Caprylic/capric triglycerides | 9% |
| | Olivem-800 (Ceteareth-6 Olivate) | 3% |
| | Cetyl alcohol | 2% |
| | Oliwax LC(Cetyl Palmitate, Sorbitan Palmitate, Sorbitan Olivate) | 2% |
| | Enriched *P. lanoso-viride* 8D glycoprotein fraction | 1,5% (10 mg/ml) |
| | Ethanol | 0,75% |
| | Tocopherol acetate | 0,5% |
| | Potassium sorbate | 0,4% |
| | Gluconolactone, Sodium Benzoate | 0,4% |

| | | |
|---|---|---|
| Mixture a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and the formulation is homogenized. | | |

**Example 3 - eye serum containing glycoprotein enriched fraction from P.lanoso-viride 8D**

| | | |
|---|---|---|
| a) | Aqua | 71,42% |
| b) | Squalane | 10% |
| | Coco-caprylate | 10% |
| | Olivem-100 (Cetearyl Olivate, Sorbitan Olivate) | 3% |
| | Cetyl alcohol | 2% |
| | Enriched *P. lanoso-viride* 8D glycoprotein fraction | 1,5% (10 mg/ml) |
| | Ethanol | 0,75% |
| | Tocopherol acetate | 0,5% |
| | Potassium sorbate | 0,4% |
| | Gluconolactone, Sodium Benzoate | 0,4% |
| | Ubiquinone | 0,03% |

| | | |
|---|---|---|
| Ingredient a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and formulation is homogenized. | | |

**Example 4 - regenerating cream containing purified glycoprotein fraction (AMPD) from P.lanoso-viride8D**

| | | |
|---|---|---|
| a) | Aqua | 54,45% |
| b) | Vitis vinifera (Grape) Seed Oil | 20% |
| | Caprylic/capric triglycerides | 10% |
| | Titanium dioxide | 6% |
| | Olivem-100 (Cetearyl Olivate, Sorbitan Olivate) | 4% |
| | Cetyl alcohol | 2% |
| | Purified *P. lanoso-viride* 8D glycoprotein fraction | 1,5% (10 µg/ml) |
| | Ethanol | 0,75% |
| | Tocopherol acetate | 0,5% |
| | Potassium sorbate | 0,4% |
| | Gluconolactone, Sodium Benzoate | 0,4% |

| | | |
|---|---|---|
| Ingredient a) is heated to approximately 65°C b) is likewise heated to approximately 65 °C till emulsifying waxes are completely melted, and then added while vigorously stirring to mixture a) to form emulsion. Stirring is continued until the cream has cooled down to approximately 35 °C. Then mixture c) is added while stirring and the cream is homogenized. | | |

**Example 5 - eye serum containing purified glycoprotein fraction (AMPD) from P.lanoso-viride8D**

| | | |
|---|---|---|
| a) | Aqua | 71,42% |
| b) | Squalane | 10% |
| | Coco-caprylate | 10% |
| | Olivem-100 (Cetearyl Olivate, Sorbitan Olivate) | 3% |
| | Cetyl alcohol | 2% |
| | Purified P. lanoso-viride 8D glycoprotein fraction | 1,5% (10 µg/ml) |
| | Ethanol | 0,75% |
| | Tocopherol acetate | 0,5% |
| | Potassium sorbate | 0,4% |
| | Gluconolactone, Sodium Benzoate | 0,4% |
| | Ubiquinone | 0,03% |

| | | |
|---|---|---|
| Ingredient a) is heated to approximately 65°C and mixture b) is likewise heated to approximately 65 °C till emulsifying waxes are completely melted, and then added while vigorously stirring to mixture a) to form emulsion. Stirring is continued until the formulation has cooled down to approximately 35 °C. Then mixture c) is added while stirring and formulation is | | |

### References

1. Antignac E, Nohynek GJ,Re T, Clouzeau J, Toutain H. 2011. Safety of botanical ingredients in personal care products/cosmetics. Food and Chemical Toxicology, 49: 324-341
2. Baschong W, Mongiat S, Ochs D. 2007. WO2007062995 A3, Glucan compositions.
3. Bolognia JL., 1993. Dermatologic and cosmetic concerns of the older woman. Clin. Geriatr. Med. 9: 209-229.
4. Bombardelli E, Ponzone C. 1996. WO1996020284 A1, Hydroxyproline-rich proteins and pharmaceutical and cosmetic formulations containing them.
5. Campisi J. 1998. The role of cellular senescence in skin aging. J Investig Dermatol Symp Proc. 3(1):1-5.
6. Chung SH, Jung MH,Kim JK,Lee JH, Lee KH,Song JH, Yoon JS. 2008. WO 2008084890 A1, Cosmetic compositions for skin-tightening and method of skin-tightening using the same.
7. Du B, Bian Z, Xu B. 2013. Skin Health Promotion Effects of Natural Beta-Glucan Derived from Cereals and Microorganisms: A Review. Phytother. Res. (2013) doi: 10.1002/ptr.4963
8. Duena AP,Garces JG,Sanchez JG,Anton JMG, Marano CM, Del Pozo MR,Coma SV. 2006. US7022668 B2, Use of a glycoprotein for the treatment and re-epithelialisation of wounds.
9. Gilchrest BA., 1989. Skin ageing and photoageing. An overview. J. Am. Acad. Dermatol. 21, 510-513.
10. Guyton K.Z., Gorospe M., Holbrook NJ., 1997. Oxidative stress, gene expression, and the aging process. In: Scandalios, J. (Ed.), Oxidative Stress and the Molecular Biology of Antioxidant Defences. Cold Spring Harbour Laboratory Press, NY, USA, pp. 247-272.
11. Jenkins G. 2002. Molecular mechanisms of skin ageing. Mechanisms of Ageing and Development, 123: 801-810
12. Kludas M. 2000. EP0668072 B1, Cosmetic composition containing a plant extracellular matrix extract.
13. Mukherjee PK, Maity N, Nema NK, Sarkar BK. 2011. Bioactive compounds from natural resources against skin aging. Phytomedicine 19: 64-73
14. Nikolajeva V, Eze D, Kamradze A, Indulena M, Muiznieks I. 1996. Protective effect of adenylate deaminase (from Penicillium lanoso-viride) against acute infections in mice. Immunopharmacology, 35 (2): 163-169.
15. Nikolajeva V, Eze D, Petrina Z, Muiznieks I. 1999. Activation of mice peritoneal machrphages by adenylate deaminase from Penicillium lanoso-viride. Proceedings of the Latvian Academy of Sciences, sect. B, 53 (1):12-15.
16. Nikolajeva V, Eze D, Petrina Z, Muiznieks I. 2000. Treatment of experimental autoimmune encephalomyelitis with adenylate deaminase from Penicillium lanoso-viride. Journal of autoimmunity,14:107-113
17. Nikolajeva V, Artjuha M, Eze D, Petrina Z, Babarikins D, Muiznieks I. LV 13959B, Adanylate deaminase containing hydrogel and its use in treatment of skin ulcers.
18. Pieroni A, Quave CL, Villanelli ML, Mangino P, Sabbatini G, Santini L, BoccettiT, Profili M, Ciccioli T, Rampa LG, Antonini G, Girolamini C , Cecchi M, Tomasi M. 2004. Ethnopharmacognostic survey on the natural ingredients used in folk in the inland Marches, Central-Eastern Italy. Journal of Ethnopharmacology, 91:331-344
19. Robert L, Labat-Robert L, Robert AM. 2009. Physiology of skin aging. Pathologie Biologie, 57:336-341
20. Shaoqiong X, Wanqing L, Zhirong Y, Zhidong W. 2008. Related gene expressions in anti-keratinocyte aging induced by Ganoderma lucidum polysaccharides. Journal of Medical Colleges of PLA 23:167-175
21. Singh RP, Agarwal R. 2009 Cosmeceuticals and silibinin. Clin Dermatol 27: 479-484.
22. Sohal RS, Weindruch R., 1996. Oxidative stress, caloric restriction and ageing. Science 273: 59-63.
23. Yingngam B, Rungseevijitprapa W. 2012. Molecular and clinical role of phytoestrogens as anti-skin-ageing agents: A critical overview. Phytopharmacology, 3(2): 227-244
24. Zak M, Novak F, Muiznieks I, Nikolajeva V, Kamradze A. 1986. Immunopotentiating and antitumor activity of the mould glycoprotein. A. Sbornik Lekarsky 88:139-145
25. Zouboulis CC, Makrantonaki E. 2011. Clinical aspects and molecular diagnostics of skin aging. Clinics in Dermatology,29: 3-14

## Claims

1. A method for the isolation of glycoprotein-rich fungal extract from sporulating mycelium of fungus *Penicillium lanoso-viride* 8D by disintegration and extraction of mycelium in phosphate buffered saline with following enrichment, wherein the sporulating mycelium for isolation of glycoprotein-rich fungal extract is cultivated on the surface of malt extract broth.

2. The method according to claim 1, **characterized in that** enriched glycoprotein fraction is isolated by precipitation at 4 °C with ammonium sulphate at 50-77% saturation.

3. The method according to claim 1 or 2, wherein purified, adenylate deaminase activity possessing glycoprotein fraction is isolated with gelfiltration of enriched glycoprotein fraction on Sephadex G-150 or G-200 with phosphate buffered saline.

4. A use of enriched glycoprotein fraction isolated according to any preceding claim in cosmetic compositions at concentrations 5-15 mg/ml for skin anti-ageing purposes.

5. The use of purified glycoprotein fraction isolated according to any preceding claim in cosmetic compositions at concentrations 5-10 µg/ml for skin anti-ageing purposes.

## Patentansprüche

1. Ein Verfahren zur Isolierung von Glykoprotein-reichem Pilzextrakt aus sporulierendem Myzel des Pilzes *Penicillium lanoso-viride* 8D durch Desintegration und Extraktion von Myzel in phosphatgepufferter Kochsalzlösung mit anschließender Anreicherung, wobei das sporulierende Myzel zur Isolierung von Glykoprotein-reichem Pilzextrakt auf der Oberfläche der Malzextraktbrühe kultiviert wird.

2. Das Verfahren nach Anspruch 1 ist **dadurch gekennzeichnet, dass** die angereicherte Glykoproteinfraktion durch Ausfällung bei 4 °C mit Ammoniumsulfat bei 50-77 % Sättigung isoliert wird.

3. Verfahren nach Anspruch 1 oder 2, wobei die gereinigte Adenylatdeaminase-Aktivität, die eine Glykoproteinfraktion besitzt, mit Gelfiltration der angereicherten Glykoproteinfraktion auf Sephadex G-150 oder G-200 mit phosphatgepufferter Kochsalzlösung isoliert wird.

4. Verwendung einer angereicherten Glykoproteinfraktion, die nach einem der vorhergehenden Ansprüche in kosmetischen Zusammensetzungen mit Konzentrationen von 5-15 mg/ml für Haut-Anti-Aging-Zwecke isoliert wird.

5. Verwendung einer gereinigten Glykoproteinfraktion, die nach einem der vorhergehenden Ansprüche in kosmetischen Zusammensetzungen mit Konzentrationen von 5-10 µg/ml für Haut-Anti-Aging-Zwecke isoliert wird.

## Revendications

1. Procédé d'isolement d'un extrait fongique riche en glycoprotéines provenant de mycélium sporulant de champignon *Penicillium lanoso-viride* 8 D par désintégration et extraction de mycélium dans une solution saline tamponnée au phosphate avec enrichissement consécutif, dans lequel le mycélium sporulant pour isolement d'extrait fongique riche en glycoprotéines est cultivé sur la surface d'un bouillon d'extrait de malt.

2. Procédé selon la revendication 1, **caractérisé en ce que** la fraction enrichie de glycoprotéines est isolée par précipitation à 4 °C avec du sulfate d'ammonium à 50-77 % de saturation.

3. Procédé selon la revendication 1 ou 2, dans lequel une fraction purifiée de glycoprotéines possédant une activité d'adénylate désaminase est isolée avec une filtration sur gel d'une fraction enrichie de glycoprotéines sur du Sephadex G-150 ou G-200 avec une solution saline tamponnée au phosphate.

4. Utilisation d'une fraction enrichie de glycoprotéines, isolée selon l'une quelconque des revendications précédentes dans des compositions cosmétiques à des concentrations de 5 à 15 mg/mL à des fins d'anti-vieillissement de la peau.

5. Utilisation d'une fraction purifiée de glycoprotéines, isolée selon l'une quelconque des revendications précédentes dans des compositions cosmétiques à des concentrations de 5 à 10 µg/mL à des fins d'anti-vieillissement de la peau.
